# EUROPEAN PATENT APPLICATION

(11) **EP 2 452 713 A1**
(43) Date of publication of application: **16.05.2012**
(21) Application number: 10797039.4
(22) Date of filing: 28.06.2010
(51) Int. Cl.: A61M 5/34, A61B 5/153

(54) **NEEDLE TUBE, MEDICAL INSTRUMENT, AND METHOD FOR MANUFACTURING MEDICAL INSTRUMENT**

(30) Priority: 06.07.2009 JP 2009159993
(71) Applicant: Terumo Kabushiki Kaisha, Tokyo 151-0072 (JP)
(72) Inventor: KAWAMOTO, Hideo, Nakakoma-gun Yamanashi 409-3853 (JP); OYAUCHI, Tetsuya, Nakakoma-gun Yamanashi 409-3853 (JP); AKIYAMA, Kouichi, Nakakoma-gun Yamanashi 409-3853 (JP)
(74) Representative: TBK
(86) International application number: PCT/JP2010/060984
(87) International publication number: WO 2011/004728

(57) **Abstract**

[Object]

To prevent a needle tube from dropping out of a holding member for holding the needle tube without executing any surface treatment on the needle tube or the holding member.

[solving Means]

A syringe needle assembly 1 has a needle tube 2 and a hub 3. The needle tube 2 has a tubular needle 4 and a enlarged portion 5. The tubular needle 4 is provided with a needlepoint which can be punctured into a living body. The enlarged portion 5 is annularly formed so as to be continuous in the circumferential direction of the tubular needle 4 by executing press working on the tubular needle 4 in the axial direction. The hub 3 has a fitting portion 38a to which the enlarged portion 5 of the needle tube 2 is fitted, and holds the needle tube 2.

## Description

### Technical Field

The present invention relates to a needle tube to be punctured into a living body, a medical instrument using the needle tube and a method for manufacturing the medical instrument.

### Background Art

Stainless steel is generally used as a material of a syringe needle (needle tube) used when medicinal solution is injected, body fluid is extracted or the like. A needlepoint which can be inserted into a living body is provided to one end of the needle tube. The needle tube as described above is fixed to a holding member such as a hub formed of resin or the like to construct a medical instrument.

A fixing method using insert molding is known as a method of fixing a needle tube to a holding member (Patent Document 1), for example. It is known that the adhesiveness between the needle tube and the holding member is enhanced by executing a surface roughening treatment such as an acid treatment, a chemical treatment such as a plasma treatment or the like, a blast treatment or the like on the outer periphery of the needle tube when insert molding is executed (Patent Document 2). A method using adhesive is also known as another method of fixing the needle tube to the holding member.

### Related Art Document

### Patent Document

Patent Document 1: JP-A-2002-315740
Patent Document 2: JP-A-2005-152363

### Summary of the Invention

### Problem to be solved by the Invention

However, according to the method using the insert molding described in the Patent Document 1, the surface of the needle tube is smooth and thus there is a concern that the needle tube is detached from the holding member. Therefore, when the adhesiveness between the needle tube and the holding member is enhanced by executing the surface treatment on the needle tube as described in the Patent Document 2, it increases the number of working steps and thus causes reduction in production efficiency.

Furthermore, even when the needle tube and the holding member are fixed to each other by using adhesive, it is necessary to subject the needle tube or the holding member to the surface treatment in order to enhance the adhesiveness of the adhesive.

The needle tube is formed of metal such as stainless or the like, and the holding member is formed of resin such as polypropylene, polyethylene or the like. With respect to general adhesive, when it has an excellent adhesive characteristic to metal, the adhesive characteristic to resin is weak, and when it has an excellent adhesive characteristic to resin, the adhesive characteristic to metal is weak. Therefore, it is necessary that a surface treatment to enhance the adhesion of adhesive being used is executed on the needle tube or the holding member in accordance with the adhesive concerned.

The present invention has been implemented in view of such a situation, and has an object to prevent a needle tube from dropping out of a holding member for holding the needle tube without executing any surface treatment on the needle tube or the holding member.

### Means of solving the Problem

A medical instrument of the present invention has a needle tube and a holding member. The needle tube has a needle body and a enlarged portion. The needle body is provided with a needlepoint which can be punctured into a living body. The enlarged portion is annularly formed so as to be continuous in the circumferential direction of the needle body by executing press working on the needle body in the axial direction. The holding member has a fitting portion to which the enlarged portion of the needle tube is fitted, and holds the needle tube.

The medical instrument manufacturing method of this invention has a press step and an instrument forming step. In the press step, the needle body provided with the needlepoint which can be punctured into the living body is subjected to press working in the axial direction to form an annular enlarged portion which is continuous in the circumferential direction of the needle body. In the instrument forming step, the holding member having a fitting portion to which the enlarged portion of the needle tube is fitted is made to hold the needle tube.

In the needle tube, the medical instrument and the medical instrument manufacturing method of this invention, the needle body is subjected to the press working in the axial direction, whereby the annular enlarged portion continuous in the circumferential direction of the needle body is formed on the needle tube. This enlarged portion of the needle tube is fitted to the fitting portion of the holding member, whereby the movement of the needle tube with respect to the holding member is stopped, and the needle tube can be prevented from dropping out of the holding member.

### Effect of the Invention

According to the needle tube, the medical instrument and the medical instrument manufacturing method of the present invention, the needle tube can be prevented from dropping out of the holding member without executing the surface treatment on the needle tube or the holding member.

### Brief Description of the Drawings

[Fig. 1] is a cross-sectional view showing a first embodiment of a medical instrument according to the present invention.
[Fig. 2] is a diagram showing a method of manufacturing a enlarged portion in the needle tube according to the first embodiment of the medical instrument of the present invention.
[Fig. 3] is a cross-sectional view of the enlarged portion according to the first embodiment of a syringe needle assembly of the present invention.
[Fig. 4] is a cross-sectional view showing a second embodiment of a medical instrument according to the present invention.
[Fig. 5] is a cross-sectional view showing a third embodiment of a medical instrument according to the present invention.
[Fig. 6] is a cross-sectional view showing a fourth embodiment of a medical instrument according to the present invention.

### Embodiments for carrying out the Invention

Best modes for carrying out a needle tube, a medical instrument and a medical instrument manufacturing method of the present invention will be described with reference to the drawings. Common members in the respective drawings are represented by the same reference numerals.
The description will be made in the following order.
1. First Embodiment
2. Second Embodiment
3. Third Embodiment
4. Fourth Embodiment

### First Embodiment

### [Example of Construction of Medical Instrument]

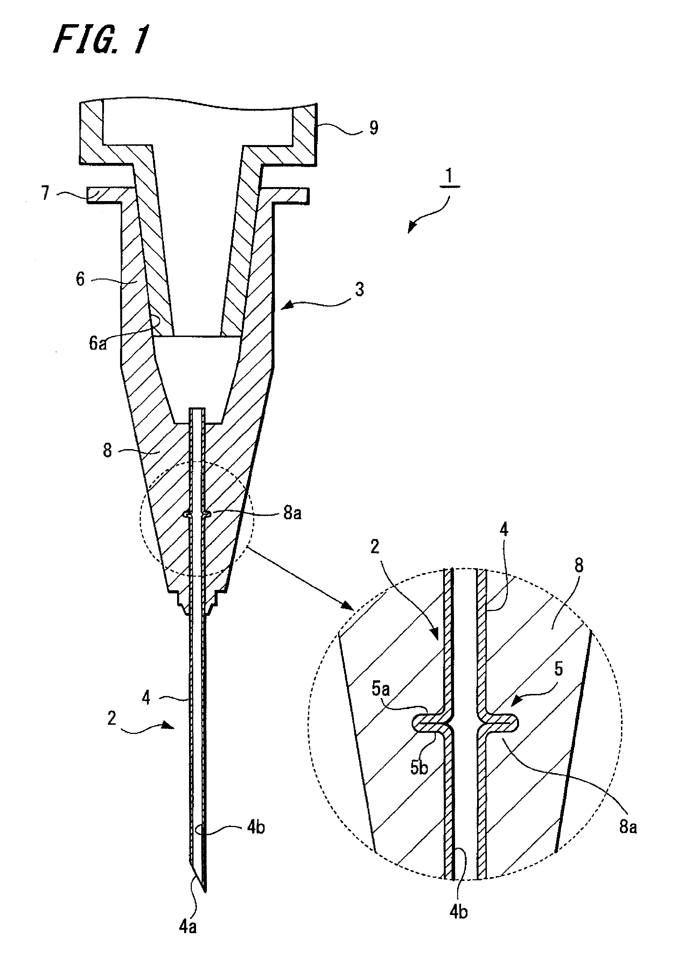

First, an example of the construction of a first embodiment of a medical instrument according to the present invention will be described with reference to Fig. 1.
Fig. 1 is a cross-sectional view showing the first embodiment of the medical instrument of the present invention.

The medical instrument 1 is a syringe needle assembly used when medicinal solution is injected, body fluid is extracted or the like. This medical instrument (hereinafter referred to as "syringe needle assembly") 1 has a hollow tubular needle 2 and a hub 3 for holding the needle tube 2. A syringe 9 represented by a broken line is connected to the hub 3 of the syringe needle assembly 1 to construct a medical agent injecting device.

### [needle tube]

The needle tube 2 has a tubular needle 4, and a enlarged portion 5 formed integrally with the tubular needle 4. With respect to the usable tubular needle 4, the size thereof ranges from 10 gauges (about 3.4mm in outer diameter) to 36 gauges (about 0.1mm in outer diameter), preferably ranges from 14 gauges (about 2.1mm in outer diameter) to 36 gauges, and more preferably ranges from 28 gauges (about 0.36mm in outer diameter) to 36 gauges. A cutting edge face 4a for making a needlepoint sharply-angled is formed at one end of the tubular needle 4.

Stainless steel may be used as the material of the tubular needle 4, for example. However, the present invention is not limited to this material, and aluminum, aluminum alloy, titan, titan alloy or other metal may be used. Not only a straight needle, but also a double-edged needle or a taper needle having at least a tapered part may be used. The taper needle may be configured so that the base end portion thereof has a larger diameter than the needlepoint end portion and the intermediate portion thereof is tapered. The cross-sectional shape of the needle tube 2 is not limited to a circular shape, and it may be a polygonal shape such as a triangle or the like.

The enlarged portion 5 is provided at any position of the tubular needle 4. The enlarged portion 5 is formed as an annular projecting portion continuous in the circumferential direction of the tubular needle 4 by executing press working on the tubular needle 4 in the axial direction. It is preferable that the diameter of the enlarged portion 5 is 1.1 time to three times as large as the outer diameter of the tubular needle 4. A method of manufacturing the needle tube 2 having this enlarged portion 5 will be described in detail later. One end face 5a of the enlarged portion 5 faces the base end side of the needle body, and the other end face 5b faces the needlepoint side. The end faces 5a and 5b of the enlarged portion 5 are flat faces which are substantially perpendicular to the axial direction of the tubular needle 4. In this embodiment, the enlarged portion 5 is provided at any position of the tubular needle 4, however, the enlarged portion 5 must be provided so as to meet a needle holding portion 8 so that the tubular needle 4 can be fixed to the needle holding portion 8 of a hub 3. The enlarged portion 5 may be provided at any position insofar as it meets the needle holding portion 8.

### [Hub]

The hub 3 represents a concrete example of the holding member for holding the needle tube 2. The hub 3 is formed of synthetic resin such as polycarbonate, polypropylene, polyethylene or the like, and it is formed integrally with the needle tube 2 by insert molding or welding.

The hub 3 is designed in a substantially hollow cylindrical shape, and it has a barrel portion 6, a flange portion 7 and the needle holding portion 8. A barrel hole 6a of the barrel portion 6 intercommunicates with a needle hole 4b of the needle tube 2. The syringe 9 is inserted from one end of the barrel portion 6 into the barrel hole 6a, and detachably connected to the hub 3. A flange portion 7 is provided to one end of the barrel portion 6. This flange portion 7 may come into contact with the syringe 9.

The needle holding portion 8 may be provided so as to be continuous with the other end of the barrel portion 6, and designed in a substantially circular truncated conical shape so as to be tapered at the tip side thereof. The needle holding portion 8 is fixed while containing the intermediate portion of the needle tube 2 (the tubular needle 4). The intermediate portion of the needle holding portion 8 serves as a fitting portion 8a which is brought into close contact with and fitted to the enlarged portion 5 of the needle tube 2. The enlarged portion 5 of the needle tube 2 is fitted to the fitting portion 8a, whereby the needle tube 2 is prevented from moving with respect to the needle holding portion 8. As a result, the needle tube 2 can be prevented from dropping out of the hub 3.

### [Syringe]

The syringe 9 may be configured to fill medical agent when the medical agent injecting device is used, or it may be a pre-filled syringe which is filled with medical agent in advance. Furthermore, vaccine may be cited as medical agent to be filled in the syringe 9, however, a material using high-molecular substance such as cytokine or the like may be used or hormone may be u sed. Furthermore, a cutting edge face may be also provided to the other end of the needle tube main body 4, whereby the needle tube 2 is designed as a double-edged needle. The other end of the needle tube 2 may be designed to penetrate through the plug body of the pre-filled syringe and intercommunicate with the medicinal solution tank.

### [Method of Manufacturing Needle Tube having Enlarged portion]

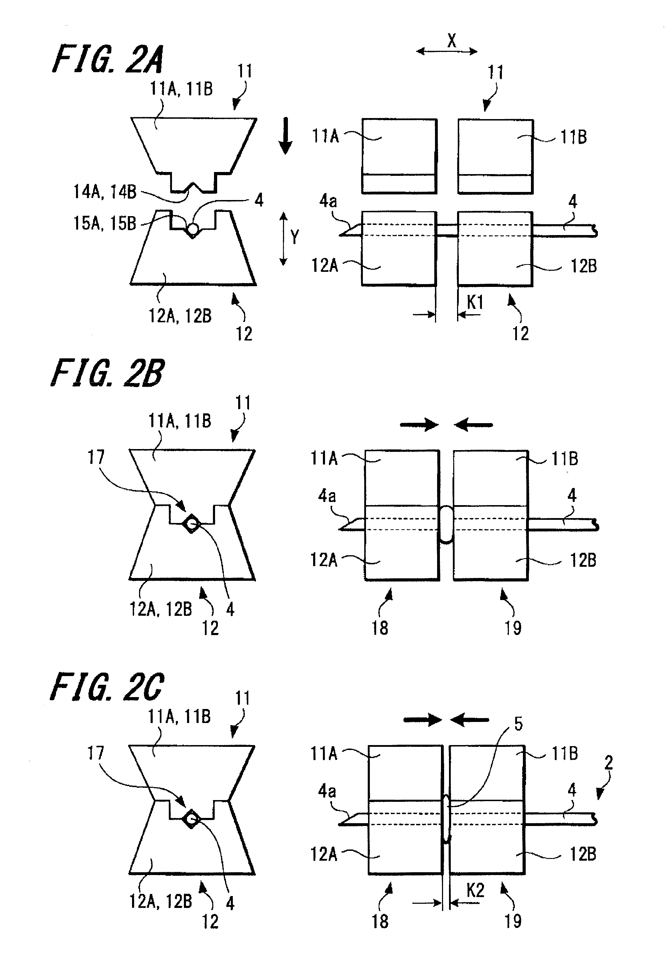

Next, a method of manufacturing the needle tube 2 having the enlarged portion 5 will be described with reference to Fig. 2.
Fig. 2A is a diagram showing a state that the needle tube 2 is sandwiched by using upper metal molds and lower metal molds. Fig. 2B is a diagram showing a state that the needle tube 2 is subjected to press working in the axial direction by using the upper metal molds and the lower metal molds. Fig. 2C is a diagram showing a state that a enlarged portion is formed by the press working.

The enlarged portion 5 is formed by subjecting the tubular needle 4 to the press working in the axial direction. A device for performing the press working has a pair of upper metal molds 11A and 11B and a pair of lower metal molds 12A and 12B.

The pair of upper metal molds 11A and 11B are provided in juxtaposition with each other in a first direction X, and are movable in the first direction X and a second direction Y perpendicular to the first direction X. A fitting groove 14A is formed on the lower surface of the upper metal mold 11A, and a fitting groove 14B is formed on the lower surface of the upper metal mold 11B. These fitting grooves 14A and 14B extend in the first direction X, and are opposed to each other in the first direction X. The cross-sectional shapes of the fitting grooves 14A and 14B are set to the same triangular shape. The fitting grooves 14A and 14B of the pair of upper metal molds 11A and 11B are fitted to the circumferential surface of the tubular needle 4.

The pair of lower metal molds 12A and 12B are provided in juxtaposition with each other in the first direction X as in the case of the pair of upper metal molds 11A and 11B. The upper surface of the lower metal mold 12A faces the lower surface of the upper metal mold 11A in the second direction Y, and the upper surface of the lower metal mold 12B faces the lower surface of the upper metal mold 11B in the second direction Y. The lower metal mold 12A moves in the first direction X together with the upper metal mold 11A. The lower metal mold 12B moves in the first direction X together with the upper metal mold 11B.

A fitting groove 15A is formed on the upper surface of the lower metal mold 12A, and a fitting groove 15B is formed on the upper surface of the lower metal mold 12B. These fitting grooves 15A and 15B extend in the first direction X, and are opposed to each other in the first direction X. The fitting groove 15A faces the fitting groove 14A of the upper metal mold 11A in the second direction Y, and the fitting groove 15B faces the fitting groove 14B of the upper metal mold 11B in the second direction Y. The fitting grooves 15A and 15B are designed to have the same triangular cross-sectional shape.

The circumferential surface of the tubular needle 4 is fitted to the fitting grooves 15A and 15B of the pair of lower metal molds 12A and 12B. That is, the tubular needle 4 is sandwiched by the upper metal mold 11A and the lower metal mold 12A, and also sandwiched by the upper metal mold 11B and the lower metal mold 12B.

In order to form the enlarged portion 5 integrally with the tubular needle 4, the interval distance between the upper metal mold 11A (the lower metal mold 12A) and the upper metal mold 11B (the lower metal mold 12B) is first set to K1 (hereinafter referred to as "distance K1"). Subsequently, the tubular needle 4 is inserted into the fitting grooves 15A and 15B of the lower metal molds 12A and 12B (see Fig. 2A).

Subsequently, the upper metal molds 11A and 11B are moved in the second direction Y, and brought into contact with the lower metal molds 12A and 12B, respectively. Accordingly, the tubular needle 4 is pinched by a first pinching portion 18 comprising the upper metal mold 11A and the lower metal mold 12A, and also pinched by a second pinching portion 19 comprising the upper metal mold 11B and the lower metal mold 12B at a position which is far away from the first pinching portion 18 by the distance K1.

At this time, the tubular needle 4 is engaged with an engaging portion 17 having a rectangular cross-section which is configured by the fitting grooves 14A and 14B and the fitting grooves 15A and 15B. That is, the tubular needle 4 is pressed and fixed to the four flat faces of the engaging portion 17. Accordingly, the tubular needle 4 can be surely fixed to the upper metal molds 11A and 11B and the lower metal molds 12A and 12B.

Subsequently, the first pinching portion 18 and the second pinching portion 19 are made to approach to each other, and the tubular needle 4 is subjected to press working in the axial direction thereof (see Fig. 2B). Accordingly, a portion of the tubular needle 4 which is located between the first pinching portion 18 and the second pinching portion 19 is swollen so as to heave in the circumferential direction.

When the interval distance between the first pinching portion 18 and the second pinching portion 19 is equal to K2 (hereinafter referred to as "distance K2"), the movement of the first pinching portion 18 and the second pinching portion 19 is stopped (see Fig. 2C). The distance K2 is shorter than the distance K1 (K1>K2). As a result, the annular enlarged portion 5 is formed so as to be continuous in the circumferential direction of the tubular needle 4.

The diameter and thickness of the enlarged portion 5 vary in accordance with the outer diameter and thickness of the tubular needle 4 and the distances K1, K2. Furthermore, a press pressure is different in accordance with the material, outer diameter and thickness of the tubular needle 4 and the distances K1, K2. That is, the size of the enlarged portion 5 can be adjusted by arbitrarily selecting the press pressure and the distances K1 and K2 for a given needle tube. For example, when a stainless needle body of 30 gauges (about 0.3mm in outer diameter and about 0.08mm in thickness) is used, the distance K1 is set to 0.5mm, the distance K2 is set to 0.16mm and press working is executed under force of about 30kN (3 tons), a enlarged portion of about 0.56mm in diameter can be formed.

The position at which the enlarged portion 5 is formed may be arbitrarily set in accordance with the positions of the first pinching portion 18 and the second pinching portion 19 with respect to the tubular needle 4.

### [Enlarged portion]

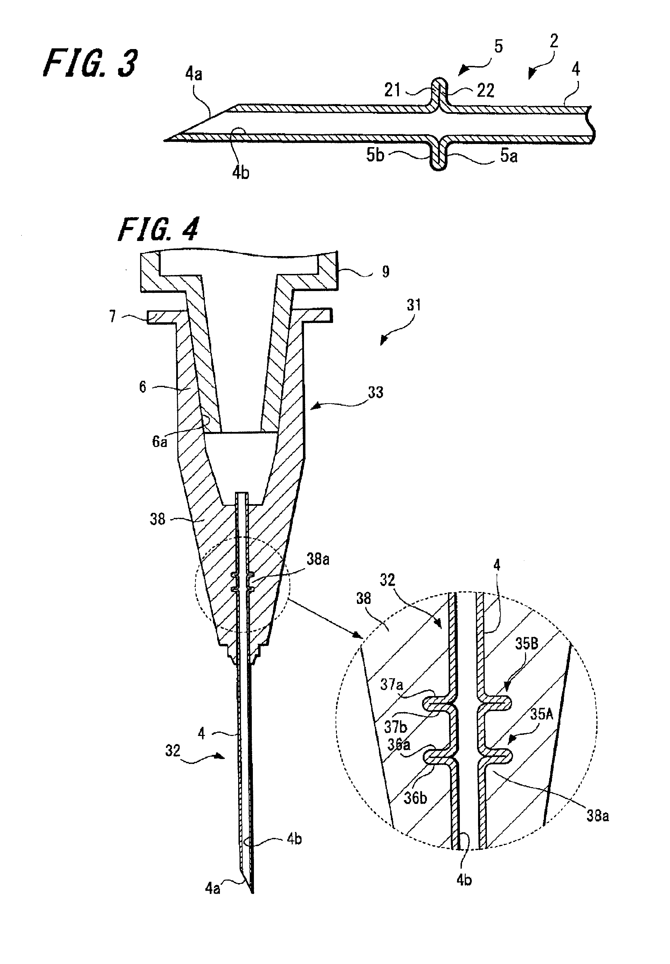

Next, the enlarged portion 5 will be described with reference to Fig. 3.
Fig. 3 is a cross-sectional view of the enlarged portion 5 in the needle tube 2.

The enlarged portion 5 comprises a first swollen piece 21 heaving in the circumferential direction of the tubular needle 4, and a second swollen piece 22 which is continuous with the first swollen piece and folded to be laminated on the first swollen piece. The first swollen piece 21 is disposed at one side of the tubular needle 4 to form the end face 5b described above. The second swollen piece 22 is disposed at the other side (hub side) of the tubular needle 4 to form the end face 5a described above.

The first swollen piece 21 and the second swollen piece 22 are laminated on each other in the axial direction of the tubular needle 4. No gap occurs between the first swollen piece 21 and the second swollen piece 22. The formation of the first swollen piece 21 and the second swollen piece 22 does not narrow the needle hole 4b of the tubular needle 4. Accordingly, even when the enlarged portion 5 is formed, there is no concern that the fluidity of medical agent passing through the needle hole 4b of the tubular needle 4 is disturbed.

### [Method of Manufacturing Medical Instrument]

Next, a method of manufacturing a syringe needle assembly 1 as a medical instrument will be described.
In the manufacturing method of the syringe needle assembly 1, a press step and an instrument forming step are executed.

In the press step, the tubular needle 4 provided with the needlepoint is subjected to press working in the axial direction to form the annular enlarged portion 5 continuous in the circumferential direction of the tubular needle 4. The press step is described in detail with respect to the method of manufacturing the needle tube having the enlarged portion described above, and thus the description thereof is omitted.

In the instrument forming step, the hub 3 as the holding member is made to hold the needle tube 2. In this embodiment, the needle tube 2 and the hub 3 are formed integrally with each other by insert molding or welding, whereby the enlarged portion 5 of the needle tube 2 is fitted to the fitting portion 8a of the needle holding portion 8. The fitting position between the enlarged portion 5 and the fitting portion 8a of the needle holding portion 8 may be arbitrarily selected. However, it is preferably set to a position at which the insert molding can be performed, and it is more preferably set to a position at which the fitting is stronger. As a result, the needle tube 2 can be prevented from dropping out of the hub 3. Accordingly, the needle tube can be prevented from dropping out of the holding member without executing the surface treatment to enhance the adhesiveness between the needle tube 2 and the hub 3.

### 2. Second Embodiment

### [Example of Construction of Medical Instrument]

Next, an example of the construction of a second embodiment of a medical instrument according to the present invention will be described with reference to Fig. 4.
Fig. 4 is a cross-sectional view showing the second embodiment of the medical instrument according to the present invention.

A medical instrument 31 is a syringe needle assembly used when medicinal solution is injected, body fluid is extracted or the like. This medical instrument (hereinafter referred to as "syringe needle assembly") 31 has the same construction as the syringe needle assembly 1 (see Fig. 1) of the first embodiment.

The difference of the syringe needle assembly 31 from the syringe needle assembly 31 resides only in two enlarged portions 35A and 35B of a needle tube 32 and a needle holding portion 38 of a hub 33. Therefore, in the following description, the two enlarged portions 35A and 35B and the needle holding portion 38 will be described, the common parts to the syringe needle assembly 1 are represented by the same reference numerals, and the description thereof is omitted.

The two enlarged portions 35A and 35B of the needle tube 32 are disposed at an intermediate portion of the tubular needle 4 so as to be spaced from each other at a predetermined interval. The interval between the enlarged portion 35A and the enlarged portion 35B may be arbitrarily set. The minimum distance of the gap between the enlarged portion 35A and the enlarged portion 35B is determined by the length of the fitting grooves 14A, 14B, 15A, 15B of the first pinching portion 18 or the second pinching portion 19.

The two enlarged portions 35A and 35B are formed as annular projecting portions continuous in the circumferential direction of the tubular needle 4 by executing the press working on the tubular needle 4 in the axial direction as in the case of the enlarged portion 5 of the first embodiment. This press working comprises a first press working for forming the enlarged portion 35A and a second press working for forming the enlarged portion 35B, for example.

One end face 36a of the enlarged portion 35A faces the base end side of the needle body, and the other end face 36b faces the needlepoint side. One end face 37a of the enlarged portion 35B faces the base end side of the needle body, and the other end face 37b faces the needlepoint side and opposes the end face 36a of the enlarged portion 35A. The end faces 36a, 36b, 37a, 37b of the enlarged portions 35A and 35B are flat faces which are substantially perpendicular to the axial direction of the tubular needle 4.

The needle holding portion 38 of a hub 33 is provided to be continuous with the other end of the barrel portion 6, and designed in a substantially circular truncated conical shape so as to be tapered. The needle holding portion 38 is fixed while containing an intermediate portion of the needle tube 32 (tubular needle 4). An intermediate portion of the needle holding portion 38 serves as a fitting portion 38a which is brought into close contact with and fitted to the enlarged portions 35A and 35B of the needle tube 32. The fitting of the enlarged portions 35A and 35B of the needle tube 32 to the fitting portion 38a prevents movement of the needle tube 32 relatively to the needle holding portion 38. As a result, the needle tube 32 can be prevented from dropping out of the hub 33.

In the syringe needle assembly 31, the enlarged portions 35A and 35B of the needle tube 32 are fitted to the fitting portion 38a of the hub 33. That is, two retaining members for the needle tube 32 are provided. Therefore, the resistance force to the drop of the needle tube 32 from the hub 33 can be increased, and the drop preventing effect of the needle tube 32 can be enhanced. The needle tube according to this embodiment may be configured to be provided with three or more enlarged portions.

The method of manufacturing the syringe needle assembly 31 is the same as the method of manufacturing the syringe needle assembly 1 described above, and thus the description thereof is omitted.

### 3. Third Embodiment

### [Example of Construction of Medical Instrument]

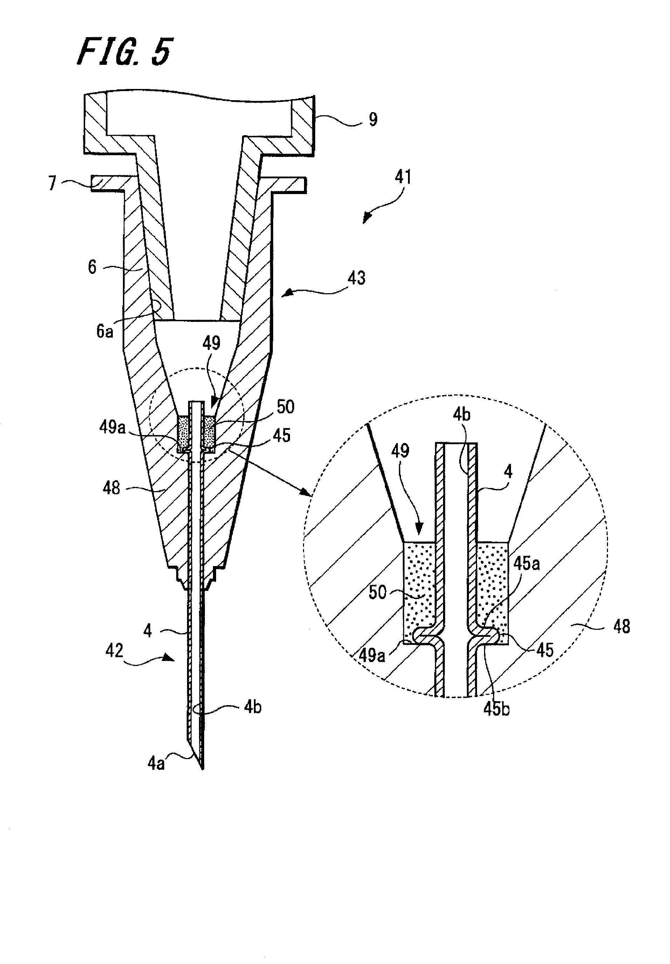

Next, an example of the construction of a third embodiment of the medical instrument according to the present invention will be described with reference to Fig. 5.
Fig. 5 is a cross-sectional view showing the third embodiment of the medical instrument of the present invention.

A medical instrument 41 is a syringe needle assembly used when medicinal solution is injected, body fluid is extracted or the like. The medical instrument (hereinafter referred to as "syringe needle assembly") 41 has the same construction as the syringe needle assembly 1 (see Fig. 1) of the first embodiment.

The difference of the syringe needle assembly 41 from the syringe needle assembly 1 resides only in a enlarged portion 45 of a needle tube 42 and a needle holding portion 48 of a hub 43. Therefore, in the following description, the enlarged portion 45 and the needle holding portion 48 will be described, and common parts to the syringe needle assembly 1 are represented by the same reference numerals, and the duplicative description is omitted.

The enlarged portion 45 of the needle tube 42 has the same shape as the enlarged portion 5 according to the first embodiment, and has end faces 45a and 45b. The difference of the enlarged portion 45 from the enlarged portion 5 resides only in the position at which it is provided to the tubular needle 4. The enlarged portion 45 is provided at a position which is displaced from the intermediate portion of the tubular needle 4 to the base end side (at the opposite side to the needlepoint).

As in the case of the hub 3 according to the first embodiment, the hub 43 is formed in a substantially hollow cylindrical shape, and it has a barrel portion 6, a flange portion 7 and a needle holding portion 48. A needle tube 42 which is provided as a separate body is fixed to the hub 43. Synthetic resin such as polycarbonate, polypropylene, polyethylene or the like may be used as the material of the hub 43, or metal such as stainless, aluminum or the like may be used.

The needle holding portion 48 of the hub 43 is provided so as to be continuous with the other end of the barrel portion 6, and it is formed in a substantially circular truncated conical shapes so as to be tapered. An intermediate portion of the needle tube 42 penetrates through the needle holding portion 48. A fitting recess portion 49 intercommunicating with a barrel hole 6a of the barrel portion 6 is provided to the needle holding portion 48. A bottom surface 49a of the fitting recess portion 49 is a flat face which is substantially perpendicular to the axial direction of the hub 43. An end face 45b of the enlarged portion 45 provided to the needle tube 42 is fitted to the bottom surface 49a of the fitting recess portion 49.

The needle tube 42 is fixed to the hub 43 by coating the fitting recess portion 49 with adhesive 50 under the state that the needle tube 42 penetrates through the needle holding portion 48. Adhesive having an excellent adhesion characteristic to the hub 43 is used as the adhesive 50. Cyanoacrylate, epoxy resin, photocurable resin or the like may be used as the adhesive 50, however, the adhesive 50 may be generated from other resin materials.

The end face 45b of the enlarged portion 45 is fitted to the bottom surface 49a of the fitting recess portion 49, whereby the needle tube 42 is prevented from moving so as to drop out of the tip portion of the needle holding portion 48. By coating the fitting recess portion 49 with the adhesive 50, the needle tube 42 is prevented from moving in all the directions other than the direction in which the needle tube 42 drops out of the tip of the needle holding portion 48. As a result, the needle tube 42 can be prevented from dropping out of the hub 43.

Even when the adhesion force of the adhesive 50 to the needle tube 42 is weak, the enlarged portion 45 of the needle tube 42 is fitted to the hardened adhesive 50, so that the movement of the needle tube 42 is prevented. Accordingly, the needle tube 42 can be prevented from dropping out of the hub 43 without subjecting the needle tube 42 or the hub 43 to the surface treatment for enhancing the adhesiveness of the adhesive.

### [Method of Manufacturing Medical Instrument]

A method of manufacturing a syringe needle assembly 41 as a medical instrument will be described.
In the method of manufacturing the syringe needle assembly 41, the press step and the instrument forming step are executed as in the case of the method of manufacturing the syringe needle assembly 1.

In the press step, the tubular needle 4 provided with the needlepoint is subjected to press working in the axial direction to form an annular enlarged portion 45 continuous in the circumferential direction of the tubular needle 4. The press step is described in detail with reference to the method of manufacturing the needle tube having the enlarged portion described above, and thus the description thereof is omitted.

In the instrument forming step, the hub 43 as the holding member is made to hold the needle tube 42. In this embodiment, the intermediate portion of the needle tube 42 is made to penetrate through the needle holding portion 48 of the hub 43, and the end face 45b of the enlarged portion 45 is fitted to the bottom surface 49a of the fitting recess portion 49. Subsequently, the fitting recess portion 49 of the hub 43 is coated with the adhesive 50, and the needle tube 42 is fitted to the needle holding portion 48.

Accordingly, the movement of the needle tube 42 with respect to the needle holding portion 48 is prevented. Therefore, the needle tube 42 can be prevented from dropping out of the hub 43 without subjecting the needle tube 42 or the hub 43 to the surface treatment for enhancing the adhesiveness of the adhesive.

### 4. Fourth Embodiment

### [Example of Construction of Medical Instrument]

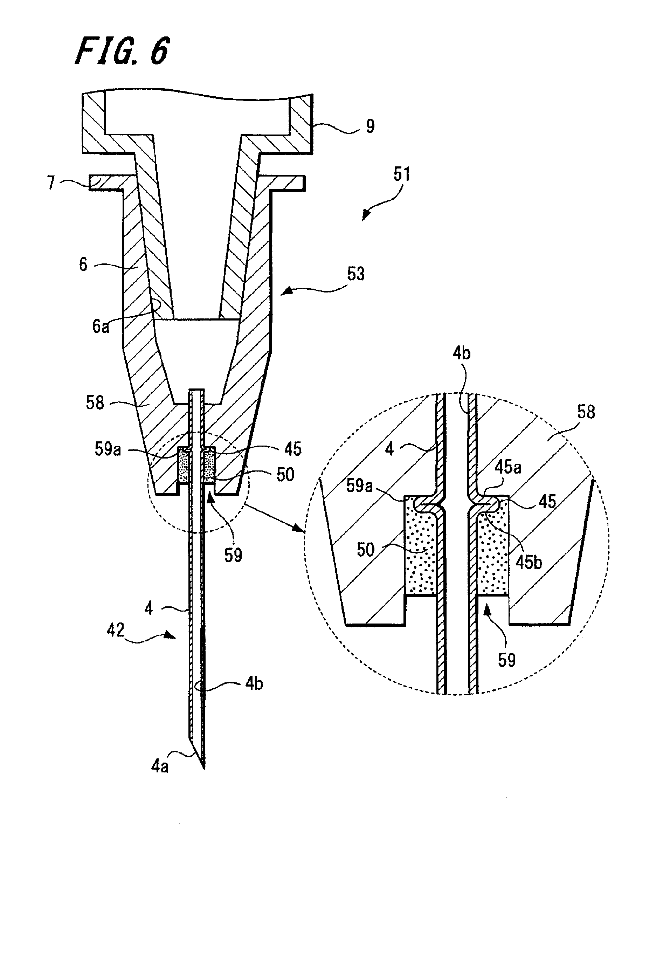

Next, an example of the construction of a fourth embodiment of the medical instrument according to the present invention will be described with reference to Fig. 6.
Fig. 6 is a cross-sectional view of a fourth embodiment of the medical instrument according to the present invention.

A medical instrument 51 is a syringe needle assembly used when medicinal solution is injected, body fluid is extracted or the like. This medical instrument (hereinafter referred to as "syringe needle assembly") 51 has the same construction as the syringe needle assembly 41 (see Fig. 1) of the third embodiment.

The difference of the syringe needle assembly 51 from the syringe needle assembly 41 resides only in a needle holding portion 58 of a hub 53. Therefore, in the following description, the needle holding portion 58 will be described. The common parts to the syringe needle assembly 41 are represented by the same reference numerals, and the duplicative description is omitted.

The hub 53 has a barrel portion 6, a flange portion 7 and a needle holding portion 58. The needle tube 42 which is provided as a separate body is fixed to the hub 53. Synthetic resin such as polycarbonate, polypropylene, polyethylene or the like may be used as the material of the hub 53, and metal such as stainless, aluminum or the like may be used.

The needle holding portion 58 of the hub 53 is provided so as to be continuous with the other end of the barrel portion 6, and designed in a substantially circular truncated conical shape so as to be tapered. An intermediate portion of the needle tube 42 penetrates through the needle holding portion 58. The needle holding portion 58 is provided with a fitting recess portion 59 which is opened to the tip face side of the needle holding portion 58. A bottom surface 59a of the fitting recess portion 59 is a flat face which is substantially perpendicular to the axial direction of the hub 53. The end face 45a of the enlarged portion 45 provided to the needle tube 42 is fitted to the bottom surface 59a of the fitting recess portion 59.

The needle tube 42 is fixed to the hub 53 by coating the fitting recess portion 59 with the adhesive 50 under the state that the needle tube 42 penetrates through the needle holding portion 58. At this time, the end face 45a of the enlarged portion 45 is fitted to the bottom surface 59a of the fitting recess portion 59, whereby the needle tube 42 is prevented from moving so as to drop out of the rear end (the barrel portion 6 side) of the needle holding portion 58. Accordingly, the needle tube 42 can be surely prevented from dropping (releasing) out of the hub 53 due to resistance applied to the needle tube when the needle tube 42 is made to sting into a skin or the like.

### [Method of Manufacturing Medical Instrument]

Next, a method of manufacturing a syringe needle assembly 51 as a medical instrument will be described.
In the method of manufacturing the syringe needle assembly 51, the press step and the instrument forming step are executed as in the case of the method of manufacturing the syringe needle assembly 1. The press step is described in detail with reference to the method of manufacturing the needle tube having the enlarged portion described above, and thus the description thereof is omitted hereunder.

In the instrument forming step, the hub 53 as a holding member is made to hold the needle tube 42. In this embodiment, the other end portion of the needle tube 42 is inserted from the tip side of the needle holding portion 58, and the end face 45a of the enlarged portion 45 is fitted to the bottom surface 59a of the fitting recess portion 59. Subsequently, the fitting recess portion 59 of the hub 53 is coated with the adhesive 50 to fix the needle tube 42 to the needle holding portion 58.

Accordingly, the movement of the needle tube 2 with respect to the needle holding portion 58 is prevented. At this time, even when the adhesion force of the adhesive 50 to the needle tube 42 is weak, the enlarged portion 45 of the needle tube 42 is fitted to the hardened adhesive 50, and thus the movement of the needle tube 42 is prevented. Accordingly, the needle tube 42 can be prevented from dropping out of the hub 53 without subjecting the needle tube 42 or the hub 53 to the surface treatment for enhancing the adhesiveness of the adhesive.

The needle tube, the medical instrument and the medical instrument manufacturing method according to the present invention has been described while adding the action and effect thereof. However, the needle tube, the medical instrument and the medical instrument manufacturing method of the present invention are not limited to the above-described embodiments, and various modifications may be made without departing from the subject matter described in claims. For example, not only a general syringe needle, but also a winged intravenous injection needle (winged needle), an indwelling needle, a needle-attached pre-filled syringe pre-filled with medicinal solution, an insulin syringe needle (pen needle) or the like may be used as the syringe needle assembly. Furthermore, the medical instrument of the present invention is not limited to the syringe needle, and it may be applied to a blood sampling needle.

In the above embodiments, the syringe needle assembly in which the needle tube is fixed to the hub is described as an example of the medical instrument. However, the medical instrument of the present invention is not limited to this. It is sufficient that the medical instrument of this invention has a needle tube and a holding member for holding the needle tube. For example, the medical instrument may have a needle tube and a syringe for holding the needle tube.

### Description of Reference Numerals

1, 31, 41 ... syringe needle assembly (medical instrument), 2 ... needle tube, 3 ... hub (holding member), 4 ... needle body, 5 ... enlarged portion, 6 ... barrel portion, 7 ... flange portion, 8 ... needle holding portion, 8a ... fitting portion, 9 ... syringe, 11A, 11B ... upper metal mold, 12A, 12B ... lower metal mold, 14A, 14B, 15A, 15B ... fitting groove, 18 ... first pinching portion, 19 ... second pinching portion, 21 ... first swollen piece, 22 ... second swollen piece

## Claims

1. A medical instrument, **characterized by** comprising:
a needle tube having a needle body provided with a needlepoint that can be punctured into a living body, and an annular enlarged portion that is formed by subjecting the needle body to press working in an axial direction and is continuous in a circumferential direction of the needle body; and
a holding member that has a fitting portion to which the enlarged portion is fitted, and holds the needle tube.

2. The medical instrument according to claim 1, wherein the outer diameter of the needle body ranges from 10 gauges to 36 gauges.

3. The medical instrument according to claim 1 or 2, wherein the enlarged portion has a first swollen piece heaving in a circumferential direction of the needle body, and a second swollen piece that is continuous with the first swollen piece and folded to be laminated on the first swollen piece.

4. The medical instrument according to any one of claims 1 to 3, wherein an end face at the needlepoint side of the enlarged portion is a flat face that is substantially perpendicular to an axial direction of the needle body.

5. The medical instrument according to any one of claims 1 to 4, wherein the needle tube has two or more enlarged portions.

6. A method of manufacturing a medical instrument, **characterized by** comprising:
a press step for executing press working in an axial direction on a needle body provided with a needlepoint that can be punctured into a living body, thereby forming an annular enlarged portion that is continuous in a circumferential direction of the needle body; and
an instrument forming step in which a holding member having a fitting portion to which the enlarged portion of the needle tube is fitted is made to hold the needle tube.

7. The medical instrument manufacturing method according to claim 6, wherein the press step pinches the needle body by a first pinching portion and a second pinching portion that are disposed so as to be spaced from each other at a predetermined interval in the axial direction of the needle body, and makes the first pinching portion and the second pinching portion approach to each other in the axial direction of the needle body to form the enlarged portion.

8. The medical instrument manufacturing method according to claim 6 or 7, wherein the instrument forming step forms the holding member and the needle tube integrally with each other.

9. The medical instrument manufacturing method according to claim 6 or 7, wherein the instrument forming step fixes the needle tube to the holding member by using adhesive.

10. A needle tube, **characterized by** comprising:
a needle body provided with a needlepoint that can be punctured into a living body; and
an annular enlarged portion that is formed by executing press working on the needle body in an axial direction, and is continuous in a circumferential direction of the needle body.
